# EUROPÄISCHE PATENTSCHRIFT

(11) **EP 0 804 150 B1**
(45) Veröffentlichungstag und Bekanntmachung des Hinweises auf die Patenterteilung: **23.04.2003**
(21) Anmeldenummer: 96900582.6
(22) Anmeldetag: 11.01.1996
(51) Int. Cl.: A61K 7/48

(54) **FETTSÄUREDERIVATE ENTHALTENDE LOTIONEN**
LOTIONS CONTAINING FATTY ACID DERIVATIVES
LOTIONS CONTENANT DES DERIVES D'ACIDE GRAS

(30) Priorität: 18.01.1995 DE 19501288
(43) Veröffentlichungstag der Anmeldung: 05.11.1997
(73) Patentinhaber: Beiersdorf Aktiengesellschaft, 20245 Hamburg (DE)
(72) Erfinder: SATTLER, Henning, D-22297 Hamburg (DE); ZELLE, Dagmar, D-21640 Bliedersdorf (DE)
(86) Internationale Anmeldenummer: EP9600102
(87) Internationale Veröffentlichungsnummer: WO96022079

(56) Entgegenhaltungen:
- EP-A- 0 540 857
- EP-A- 0 541 830
- WO-A-92/16184
- FR-A- 2 569 346
- US-A- 5 047 232
- PATENT ABSTRACTS OF JAPAN vol. 009, no. 218 (C-301), 5.September 1985 & JP 60 078907 A (KOBAYASHI KOOSEE:KK), 4.Mai 1985,

## Beschreibung

Gegenstand der Erfindung sind Lotionen mit einem Gehalt an Derivaten ungesättigter Fettsäuren.

Aus der WO-A-9 216 184 sind Emulsionen bekannnt , welche unter anderem mit 40 Ethylen oxideinheiten ethoxyliertes Rizinusöl enthalten.

Lotionen bieten gegenüber anderen topischen Zubereitungen den Vorteil, daß sie besonders bei großflächiger Anwendung leichter aufzutragen und zu verteilen sind und besser in die Haut einziehen als Cremes oder Salben. Besonders gilt dies für Lotionen vom W/O-Typ. Sie sind vom Emulsionstyp her für trockene Hautzustände geeignet. Außerdem ist die Bioverfügbarkeit von inkorporierten Substanzen meist besser als aus wasserfreien topischen Zubereitungen und häufig auch besser als aus O/W-Lotionen.

W/O-Lotionen sind jedoch schwierig zu stabilisieren. In Gegenwart größerer Anteile an Estern höherer Fettsäuren neigen sie zur Trennung in Fettphase und Wasserphase während der Lagerung.

Beim Einsatz von ungesättigten Fettsäurederivaten mit einer oder mehreren Doppelbindungen in solchen Lotionen kommt hinzu, daß eine stärkere Oxidationsempfindlichkeit besteht, als bei der Inkorporation in wasserfreie topischen Zubereitungen.

W/O-Lotionen mit hohem Gehalt an ungesättigen Fettsäuren bzw. deren Derivaten und guten Anwendungs-Eigenschaften, die sowohl galenisch stabil, als auch bei der Lagerung gegenüber oxidativen Einflüssen weitgehend unenpfindlich sind, lassen sich nur schwer realisieren. Dies trifft vor allem bei höheren Gehalten an diesen Fettsäuren, bzw. deren Derivaten zu.

Lotionen, in denen die ungesättigten Fettsäuren einem stärkeren Oxidations-Effekt unterliegen, sind für die topische Anwendung ungeeignet. Dies trifft vor allem bei der Verwendung an vorgeschädigter und kranker Haut zu, wie dies z.B. bei Atopikern und Psoriatikern der Fall ist. Aber auch schon bei trockenen Hautzuständen wirken sich die Oxidations-Zersetzungsprodukte, wie Peroxide, Aldehyde und Ketone negativ aus und machen den Nutzen derartiger Präparate zunichte. Besonders bemerkbar macht sich der oxidative Einfluß von Sonnenlicht.

Die Aufgabe der Erfindung bestand darin, Wasser-in-ÖI-Lotionen (W/O-Typ) zu schaffen, die stabil sind und in denen gleichzeitig die ungesättigten Fettsäuren dem schädlichen oxidativen Abbau nicht, oder nur in geringem, nichtstörendem Maße unterliegen.

Die Aufgabe der Erfindung wurde dadurch gelöst, daß W/O-Emulgatoren und Kombinationen mit anderen Emulsions-Inhaltsstoffen gefunden wurden, die sowohl die Lotion in Gegenwart hoher Anteile ungesättigter Fettsäuren bzw. deren Derivaten stabilisierten, als auch die ungesättigten Fettsäuren oxidativ stabil hielten.

Gegenstand der Erfindung sind W/O-Lotionen mit einem Gehalt an mindestens einem ungesättigten Fettsäurederivat, die dadurch gekennzeichnet sind, daß sie W/O-Emulgatoren, W/O-Stabilisatoren und/oder W/O-Emulsionssysteme, ausgewählt aus der Gruppe, bestehend aus
(A) einem W/O-Emulgator auf der Basis von ethoxyliertem Ricinusöl mit sieben Ethylenoxideinheiten
(B) einem W/O-Emulgator (B1) auf der Basis von Sorbitanisostearat und einem W/O- Stabilisator (B2) auf der Basis von Polyalkylenglykol-Copolymerisat und
(C) einem W/O-Emulsionssystem auf der Basis von Methoxy-Polyalkylenglykol-Copolymerisat, wobei entweder
   (1) der W/O-Emulgator von (A) verwendet wird, oder
   (2) der W/O-Emulgator (B1) zusammen mit dem W/O-Stabilisator B2 (B) verwendet wird, oder
   (3) eine Kombination, bestehend aus den Emulgatoren (A) und (B) mit (B1) und (B2), verwendet wird, wobei jeweils
   (4) zusammen mit (1) oder (2) oder (3) noch das Emulsionssystem (C) verwendet werden kann, und Wasser sowie gegebenenfalls Zusatzstoffe oder Hilfsstoffe enthalten.

Als W/O-Emulgator (A) wird ein ethoxyliertes hydriertes Ricinusöl verwendet, das sieben Ethylenoxideinheiten enthält. Solche Produkte sind z.B. unter der CTFA-Bezeichnung "PEG-7 Hydrogenated Castor Oil" bekannt und unter der Handelsbezeichnung "Cremophor WO 7" (BASF, DE) oder der Bezeichnung "Arlacel 989" (Atlas Chemie, ICI, DE) erhältlich.

Als W/O-Emulgator (B1) werden vorzugsweise Polyethylen- oder Polyethylenglykol-1-Glycerinsorbitanisostearate verwendet oder auch unter der CTFA-Bezeichnung "Sorbitan Isostearate + PEG-2 Hydrogenated Castor Oil + Ozokerite + Hydrogenated Castor Oil" bekannte Produkte. Besonders geeignet ist ein unter der Bezeichnung "Arlacel 582" (Atlas Chemie, ICI, DE) bekanntes Handelsprodukt.

Als W/O-Stabilisator (B2) wird ein Polyethylenglykol-Dodecylglykolcopolymer mit insbesondere 45 Ethylenglykoleinheiten bevorzugt. Solche Stabilisatoren sind auch unter der CTFA-Bezeichnung "PEG-45/Dodecyl Glycol Copolymer" bekannt. Bevorzugt wird ein unter der Handelsbezeichnung "Elfacos ST 9" (Akzo Chemie GmbH, DE) vertriebenes Produkt.

Als W/O-Emulsionssystem (C) wird ein Methoxy-Polyethylen-Dodecylglykol-Copolymer mit insbesondere 22 Ethylenglykol-Einheiten bevorzugt. Es ist unter der CTFA-Bezeichnung "Methoxy PEG-22/Dodecyl Glycol Copolymer" bekannt und als Handelsprodukt mit der Bezeichnung "Elfacos E 200" (Akzo Chemie GmbH, DE) erhältlich. Bevorzugt werden W/O-Lotionen gemäß der Erfindung mit einem Gehalt an (C).

Der Anteil der Emulgatoren, Stabilisatoren und Emulsionssysteme kann 0,5 bis 20 Gew.-% betragen, bezogen auf das Gesamtgewicht der Lotion.

Werden die Emulgatoren (A) oder (B) einzeln verwendet, werden sie vorzugsweise in Mengen von (A) 3 bis 12 Gew.-% oder (B1) von 0,5 bis 5 und (B2) 0,2 bis 5 Gew.-%, jeweils bezogen auf das Gesamtgewicht der Lotion.

Die gegebenenfalls z.B. als Emulsionssystem zugesetzte Komponente (C) liegt, dann bevorzugt in Mengen von 0,1 bis 5 Gew.-% vor, auch jeweils bezogen auf das Gesamtgewicht der Lotion.

Vorzugsweise werden die erfindungsgemäßen Emulgatoren gemeinsam verwendet, bevorzugt auch zusammen mit dem Emulsionssystem (C), und zwar insbesondere in den folgenden Gewichtsmengen, jeweils bezogen auf das Gesamtgewicht der Zubereitungen bzw. Lotionen:

| | Gew.-% | | Gew.-% |
|---|---|---|---|
| (A) | 0,1 - 10, | insbesondere | 0,5 - 5 |
| (B1) | 0,1 - 10, | insbesondere | 0,5 - 5 |
| (B2) | 0,1 - 10, | insbesondere | 0,2 - 5 |
| (C) | 0,1 - 10, | insbesondere | 0,1 - 5 |

Die erfindungsgemäßen Lotionen können ein Derivat oder mehrere Derivate ungesättigter Fettsäuren enthalten.

Das erfindungsgemäße Fettsäurederivat enthält mindestens eine ungesättigte Fettsäure. Es kann aber auch mehrere ungesättigte Fettsäuren enthalten. Triglyceride können z.B. eine, zwei oder drei ungesättigte Fettsäuren enthalten.

Erfindungsgemäß geeignete Fettsäuren der Fettsäurederivate sind einfach oder mehrfach ungesättigte Carbonsäuren mit vorzugsweise 16-24 Kohlenstoffatomen und insbesondere 1 bis 6 Doppelbindungen, besonders bevorzugt mit einer Doppelbindung oder zwei oder drei Doppelbindungen.

Die ungesättigten Fettsäuren können sowohl der n-6-Serie als auch der n-3-Serie angehören. Bevorzugt werden Fettsäuren der n-6-Serie.

Bevorzugte Derivate der Fettsäuren sind die Ester, z.B. die Glyceride und die Ester, insbesondere die Triglyceride oder die Ester mit Alkoholen, insbesondere Monoalkanolen mit vorzugsweise 1 bis 5 Kohlenstoffatomen, besonders bevorzugt die Ethylester.

Die Verbindungen können sowohl synthetisch hergestellt als auch in natürlich vorkommender Form, z.B. als Pflanzenöle eingesetzt werden.

Besonders bevorzugt werden Derivate, die ungesättigte Carbonsäuren der Kettenlänge C₁₆ - C₁₈ mit einer Doppelbindung, Carbonsäuren der Kettenlänge C₁₆ - C₂₀ mit 2 Doppelbindungen, Carbonsäuren der Kettenlänge C₁₈ - C₂₀ mit 3 Doppelbindungen und Carbonsäuren der Kettenlänge C₂₀ - C₂₂ mit 5 oder 6 Doppelbindungen enthalten. Die Doppelbindungen können sowohl isoliert als auch konjugiert vorliegen. Besonders bevorzugte Fettsäuren mit einer Doppelbindung sind Ölsäure und Palmitoleinsäure. Bei den Fettsäuren mit 2 Doppelbindungen sind besonders bevorzugt die Linolsäure, bei denen mit 3 Doppelbindungen die alpha- und gamma-Linolensäure, die Dihomo-gammalinolensäure und Arachidonsäure und bei denen mit 5 oder 6 Doppelbindungen die Eicosapentaensäure und Docosahexaensäure.

Als Ester sind besonders bevorzugt die Triglyceride und der Ethylester, insbesondere für die namentlich genannten Fettsäuren.

Die Triglyceride können sowohl 3 einheitliche, gleiche Fettsäuren als auch eine oder 2 oder 3 verschiedenartige erfindungsgemäße Fettsäuren enthalten und sie können sowohl 3 einheitlich ungesättigte Fettsäuren als auch eine oder mehrere verschiedenartig ungesättigte Fettsäuren enthalten.

Besonders bevorzugte Verbindungen sind die Ethylester und Triglyceride der Linolsäure, der alpha- und gamma-Linolensäure, der Dihomogammalinolensäure sowie der Eicosapentaensäure und Docosahexaensäure, insbesondere aber der Gammalinolensäure. Die bevorzugten Öle, die die ungesättigten Fettsäuren als Triglyceride enthalten, sind Olivenöl, Sonnenblumenkernöl, Nachtkerzensamenöl, Borretschöl, Traubenkernöl, Sojabohnenöl, Erdnußöl, Weizenkeimöl, Schwarzes Johannisbeersamenöl, das Öl aus speziellen Fungi und Fischöle.

Der Anteil der erfindungsgemäßen Fettsäurederivate in den Lotionen kann z.B. 0,1 bis 30Gew.-%, vorzugsweise 0,5 bis 20 Gew.-%, insbesondere aber 1 bis 5 Gew.-% betragen, jeweils bezogen auf das Gesamtgewicht der Lotion.

Die erfindungsgemäße Lotion kann auch noch Zusatzstoffe oder Hilfsstoffe enthalten.

Die erfindungsgemäßen Lotionen zeichnen sich durch eine geringe Oxidationsempfindlichkeit aus, so daß Zusätze von Antioxidantien nicht zwingend erforderlich sind. Werden dennoch solche Zusätze gewünscht, können die verwendeten Mengen klein bleiben.

Bevorzugte Antioxidantien sind Butyl-Hydroxy-Toluol, Butyl-Hydroxy-Anisol, Tert.-Butyl-Hydroxy-Chinon, Ascorbinsäure und ihre Derivate wie Ascorbylpalmitat, Mg-Ascorbylphosphat, Tocopherole und ihre Derivate wie alpha-Tocopherylacetat, Gallate wie Propylgallat, Octylgallat, Dodecylgallat oder andere Ester. Antioxidantien können z.B. in Mengen von 0,001 bis 5 Gew.-%, vorzugsweise 0,01 bis 1 Gew.-%, in den Lotionen enthalten sein. Weitere geeignete Antioxidantien sind t-Butylhydrochinon, Di-t-Butylhydrochinon und dl-alpha-Tocopherol.

Weiterhin können Komplexbildner, insbesondere in Kombination mit den Antioxydantien, vorzugsweise EDTA, z.B. in Gewichtsmengen von 0, 01 bis 5 Gew.-%, insbesondere 0,1 bis 1 Gew.-%, bezogen auf die gesamte Lotion enthalten sein. Weitere geeignete Komplexbildner sind Beta-Alanindiessigsäure, Hydroxyethylethylendiamintriessigsäure (Na₃HEDTA), Nitrilotriessigsäure oder Propyiendiamintetraessigsäure.

In den erfindungsgemäßen Lotionen können Fettalkohole und Mineralöle (Paraffine) enthalten sein.

Bevorzugte Fettalkohole sind Myristylalkohol, Cetylalkohol, Stearylalkohol, Cetylstearylalkohol, Behenylalkohol sowie 2-Octyldodecanol (Eutanol G).

Als Paraffin wird vorzugsweise dünnflüssiges Paraffin, gegebenenfalls auch in Kombination mit halbfestem (Vaselin) bzw. festem Paraffin und Mikrowachsen verwendet. Besonders bevorzugt werden geradkettige und verzweigtkettige Paraffine.

Die erfindungsgemäße Lotion kann zur Einstellung des pH-Wertes von z.B. pH 3 - 8, vorzugsweise pH 4 bis pH 7 Säurezusätze und Zusätze von deren Salzen in geringen Anteilen von beispielsweise etwa 0,01 - 10 Gew.-%, vorzugsweise 0,1 bis 5 Gew.-%, jeweils bezogen auf das Gesamtgewicht der Lotion, enthalten. Gut geeignet sind Carbonsäuren wie Bernsteinsäure, Milchsäure, Citronensäure oder auch Phosphorsäure und deren Salze als Puffer.

Auch Konservierungsmittel und Stabilisatoren können zugegeben werden. Geeignet sind beispielsweise Stoffe wie Parabene, Benzalkoniumchlorid, p-Chlor-m-kresol, Benzoesäure, Sorbinsäure, Phenoxyethanol, Benzylalkohol, Methyldibromglutarsäurenitril sowie deren Mischungen. Sie können in Mengen von 0,001 - 5 Gew.-%, insbesondere 0,01 bis 2 Gew.-% in der Lotion enthalten sein.

Bei den für die W/O-Lotione geeigneten Emulgatoren handelt es sich um Typen von z.T. sehr komplexer Zusammensetzungen mit niedrigen HLB-Werten sowie um deren Mischungen. Die Auswahl der Emulgatoren erfolgte unter dem Aspekt der Erzielung einer - auch bei erhöhten Temperaturen-mehrere Jahre stabilen W/O-Lotion und deren Verträglichkeit mit den Wirkstoffen bzw. der Stabilisierung der Wirkstoffe über den genannten Zeitraum.

Die Fettphase der W/O-Lotion besteht beispielsweise aus einem ausgewogenen Gemisch von flüssigen, gerad- oder verzweigtkettigen Paraffinen, natürlichen und synthetischen Estern von verzweigt- oder geradkettigen, gesättigten Fettsäuren mit ein oder mehrwertigen Alkoholen (z.B. Isopopylpalmitat, -myristat, -stearat, -isostearat, Myristylmyristat, Cetylpalmitat, mittelkettige Triglyceride, hydriertes Rizinusöl), femer Fettsäuren (z.B. Linol-, Linolensäuren, Ölsäure), Squalan und/oder Squalen, in Mengen von z.B. jeweils 0,1-30 Gew.-%, vorzugsweise 0,5 bis 10 Gew.-%, insbesondere 1 bis 5 Gew.-%, bezogen auf die gesamte Lotion. Es können die einzelnen Stoffe oder Gemische verwendet werden.

In den erfindungsgemäßen Lotionen können weitere Zusatzstoffe oder Hilfsstoffe enthalten sein.

Die Wasserphase enthält beispielsweise mehrwertige Alkohole (z.B. Glycerin, Sorbit, Propylenglycol, 1,3-Butylenglykol, Polyethylenglycole) z.B. in Mengen von jeweils 1-10 Gew.-%.

Alle Mengenangaben, Anteile und Prozentanteile sind auf das Gewicht bezogen, soweit nicht anders angegeben.

Mit der neuen erfindungsgemäßen Lotion wird eine W/O-.Lotion geschaffen, die sich durch gute Wirksamkeit, dermatologische Akzeptanz und hohe Lagerstabilität auszeichnet.

Gegenüber einer W/O-Crem bietet die W/O-Lotion den großen Vorteil, daß sie im Gegensatz zu ersterer, trotz gleichen Emulsionstyps - sich besser verteilen läßt, schneller in die Haut einzieht und keinen störenden Fettfilm hinterläßt.

Zur Herstellung der Lotion werden in an sich bekannter Weise die Bestandteile der Fettphase wie Paraffin und die Emulgatorkombination geschmolzen und auf erhöhte Temperatur, z.B. 60°C bis 80°C gebracht. Glycerin und Magnesiumsulfat und Bestandteile der Wasserphase werden bei höherer Temperatur in Wasser gelöst. Die Phasen werden vereinigt und emulgiert. Bei einer niedrigeren Temperatur, z.B. 60°-80°C, wird der Wirkstoff zugegeben. Unter Rühren läßt man die Masse abkühlen.

Aus der erfindungsgemäßen Lotion, die eine hervorragende Lagerungsstabilität aufweist, wird der Wirkstoff sehr gut absorbiert. Sie wird z.B. zur Behandlung von Atopie (z.B. Neurodermitis), Ekzemen, Dermatitis, Psoriasis sowie Entzündungen oder in der Kosmetik verwendet.

Zur Heilung oder Behandlung dieser Erkrankungen kann die erfindungsgemäße Lotion topisch auf die geschädigten Stellen aufgebracht werden. Die aufgebrachte Menge der Lotion variiert entsprechend der Konzentration des Wirkstoffs. Im allgemeinen wird eine geeignete Menge auf die geschädigte Stelle mehrmals täglich je nach Schwere der zu behandelnden Erkrankung aufgebracht.

Alle Mengenangaben, Anteile und Prozentanteile sind, soweit nicht anders angegeben , auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

Die Herstellung der W/O-Lotion geschieht folgendermaßen. Die Bestandteile der Fettphase wie Paraffin und Emulgator-(Kombination) werden in einem evakuierbaren Mischer-Homogenisator auf ca. 80°C erhitzt. Dann werden die Bestandteile der Wasserphase wie Glycerin und Magnesiumsulfat sowie Säuren bzw. Puffersubstanzen in dem Wasseranteil bei ca. 80°C gelöst, die Wasserphase wird zur Fettphase in den Mischer gegeben, und dann wird der Wirkstoff zugesetzt. Es wird Vakuum angelegt und unter Rühren und Homogenisieren auf ca. 25°C abgekühlt. Konservierungsmittel werden - in Abhängigkeit von deren physikalischen bzw. chemischen Eigenschaftenentweder in der Fettphase bei ca. 80°C oder in der Wasserphase bei ca. 80°C gelöst oder dem Ansatz bei ca. 40°C zugesetzt.

In den folgenden Beispielen werden die folgenden W/O-Emulgatoren, W/O-Stabilisatoren oder W/O-Emulsionssysteme verwendet.
(A) W/O-Emulgator ethoxyliertes, hydriertes Rizinusöl mit 7 Ethylenoxideinheiten (CTFA-Bezeichnung "PEG-7-Hydrogenated Castor Oil")
(B1) W/O-Emulgator mit der CTFA-Bezeichnung "Sorbitan Isostearate + PEG-2 Hydrogenated Castor Oil + Ozokerite + Hydrogenated Castor Oil"
(B2) W/O-Stabilisator Polyethylenglykol-Dodecylglykolcopolymer mit 45 Ethylenglykoleinheiten (CTFA-Bezeichnung "PEG-45/Dodecyl Glycol Copolymer")
(C) W/O-Emulsionssystem Methoxy-Polyethylen-Dodecylglykol-Copolymer mit 22 Ethylenglykol-Einheiten (CTFA-Bezeichnung "Methoxy PEG-22/Dodecyl Glycol Copolymer")

### Beispiel 1

Mit den angegebenen Bestandteilen wird wie oben angegeben eine W/O-Lotion hergestellt::

| | Gew.-% |
|---|---|
| Nachtkerzenöl | 22,2 |
| (C) Elfacos E 200 | 1 |
| (B1) Arlacel 582 | 2 |
| (B2) Elfacos ST 9 | 1 |
| (A) Cremophor WO 7 | 2 |
| Paraffinöl | 2,5 |
| Octyldodecanol (Eutanol G) | 5 |
| Glycerin | 3 |
| Magnesiumsulfat | 0,5 |
| Propylgallat | 0,075 |
| Phenoxyethanol | 0,5 |
| Natriumbenzoat | 0,2 |
| Benzoesäure | 0,1 |
| Wasser (dest.) | ad 100 |

### Beispiel 1a

Es wird wie in Beispiel 1 angegeben verfahren, aber statt Nachtkerzenöl wird die gleiche Gewichtsmenge Sojabohnenöl verwendet.

### Beispiel 1b

Es wird wie in Beispiel 1 angegeben verfahren, aber statt Nachtkerzenöl wird die gleiche Gewichtsmenge Sonnenblumenkernöl verwendet.

### Beispiel 1c

Es wird wie in Beispiel 1 angegeben verfahren, aber statt Nachtkerzenöl wird die gleiche Gewichtsmenge Erdnußöl verwendet.

### Beispiel 1d

Es wird wie in Beispiel 1 angegeben verfahren, aber statt Nachtkerzenöl wird die gleiche Gewichtsmenge Weizenkeimöl verwendet.

### Beispiel 1e

Es wird wie in Beispiel 1 angegeben verfahren, aber statt Nachtkerzenöl wird die gleiche Gewichtsmenge Traubenkernöl verwendet.

### Beispiel 1f

Es wird wie in Beispiel 1 angegeben verfahren, aber statt Nachtkerzenöl wird die gleiche Gewichtsmenge Olivenöl verwendet.

Die Herstellung erfolgt wie oben angegeben. Das Konservierungsmittel Phenoxyethanol wird in der Wasserphase gelöst.

### Beispiel 2

Mit den angegebenen Bestandteilen wird, wie oben angegeben, eine W/O-Lotion hergestellt:

| | Gew.-% |
|---|---|
| (A) Cremophor WO 7 | 7 |
| Paraffinöl | 9 |
| Cetearyloctanoat | 5 |
| Glycerin | 3 |
| Nachtkerzenöl | 11 |
| Magnesiumsulfat | 0,7 |
| Propylgallat | 0,05 |
| Benzylalkohol | 1 |
| Wasser | ad 100 |

### Beispiel 3

Mit den angegebenen Bestandteilen wird, wie oben angegeben, eine W/O-Lotion hergestellt:

| | Gew.-% |
|---|---|
| Nachtkerzenöl | 22,2 |
| (C) Etfacos E 200 | 0,075 |
| (B1) Arlacel 582 | 1,5 |
| (B2) Elfacos ST 9 | 0,75 |
| (A) Arlacel 989 | 1,5 |
| Paraffinöl | 2,5 |
| Octyldodecanol | 4,5 |
| Glycerin | 3 |
| Magnesiumsulfat | 0,5 |
| Propylgallat | 0,075 |
| Phenoxyethanol | 0,5 |
| Natriumbenzoat | 0,2 |
| Benzoesäure | 0,1 |
| Wasser (dest.) | ad 100 |

### Beispiel 4

Mit den angegebenen Bestandteilen wird, wie oben angegeben, eine W/O-Lotion hergestellt:

| | Gew.-% |
|---|---|
| Nachtkerzenöl | 22,2 |
| (C) Elfacos E 200 | 1 |
| (B1) Arlacel 582 | 4 |
| (B2) Elfacos ST 9 | 1,5 |
| Paraffinöl | 2,5 |
| Octyldodecanol | 4,5 |
| Glycerin | 3 |
| Magnesiumsulfat | 0,5 |
| Propylgallat | 0,075 |
| Phenoxyethanol | 0,5 |
| Natriumbenzoat | 0,2 |
| Benzoesäure | 0,1 |
| Wasser (dest.) | ad 100 |

### Beispiel 5

| | Gew.-% |
|---|---|
| Gammalinolensäureethylester | 3 |
| (C) Elfacos E 200 | 0,75 |
| (B1) Arlacel 582 | 1,5 |
| (B2) Elfacos ST 9 | 0,75 |
| (A) Cremophor WO 7 | 1,5 |
| Paraffinöl | 12,5 |
| Octyldodecanol | 5 |
| Glycerin | 3 |
| Magnesiumsulfat | 0,5 |
| Ascorbylpalmitat | 0,01 |
| Dinatrium-EDTA | 0,1 |
| Benzylalkohol | 1,5 |
| Parfum | 0,1 |

### Beispiel 6

Mit den angegebenen Bestandteilen wird, wie oben angegeben, eine W/O-Lotion hergestellt:

| | Gew.-% |
|---|---|
| Linolsäureethylester | 5 |
| (C) Elfacos E 200 | 1 |
| (B1) Arlacel 582 | 2 |
| (B2) Elfacos ST 9 | 1 |
| (A) Cremophor WO 7 | 2 |
| Paraffinöl | 2,5 |
| Caprylsäure-Caprinsäure-Triglycerid | 4,5 |
| Glycerin | 3 |
| Magnesiumsulfat | 0,5 |
| Propylgallat | 0,075 |
| Phenoxyethanol | 0,5 |
| p-Hydroxybenzoesäureethylester | 0,7 |
| Wasser (dest.) | ad 100 |

### Beispiel 7

Mit den angegebenen Bestandteilen wird, wie oben angegeben, eine W/O-Lotion hergestellt:

| | Gew.-% |
|---|---|
| Borretschöl | 10,0 |
| (B1) Arlacel 582 | 2 |
| (B2) Elfacos ST 9 | 2 |
| (A) Cremophor WO 7 | 2 |
| Paraffinöl | 10 |
| Octyldodecanol | 4,5 |
| Glycerin | 5 |
| Magnesiumsulfat | 0,5 |
| Tocopherolacetat | 0,5 |
| Phenoxyethanol | 0,5 |
| Natriumbenzoat | 0,2 |
| Benzoesäure | 0,1 |
| Wasser (dest.) | ad 100 |

### Beispiel 8

Mit den angegebenen Bestandteilen wird, wie oben angegeben, eine W/O-Lotion hergestellt:

| | Gew.-% |
|---|---|
| (A) Arlacel 989 | 7 |
| Paraffinöl | 9 |
| Octyldodecanol | 5 |
| Sorbit | 3 |
| Schwarzes Johannisbeerkernöl | 10 |
| Magnesiumsulfat | 0,7 |
| BHT | 0,1 |
| Benzylalkohol | 1 |
| Wasser | ad 100 |

### Beispiel 9

Mit den angegebenen Bestandteilen wird, wie oben angegeben, eine W/O-Lotion hergestellt:

| | Gew.-% |
|---|---|
| Traubenkernöl | 15 |
| Elfacol E 200 | 1 |
| (A) Cremophor WO 7 | 5 |
| Paraffinöl | 2,5 |
| Octyldodecanol | 4,5 |
| Propylenglycol | 5 |
| Magnesiumsulfat | 0,5 |
| Propylgallat | 0,075 |
| Phenoxyethanol | 0,5 |
| Na-Sorbat | 0,2 |
| Sorbinsäure | 0,1 |
| Wasser | ad 100 |

## Patentansprüche

1. W/O-Lotionen mit einem Gehalt an mindestens einem ungesättigten Fettsäurederivat, die **dadurch gekennzeichnet sind, daß** sie W/O-Emulgatoren, W/O-Stabilisatoren und/oder W/O-Emulsionssysteme, ausgewählt aus der Gruppe, bestehend aus
A) einem W/O-Emulgator auf der Basis von ethoxyliertem Ricinusöl mit sieben Ethylenoxideinheiten,
(B) einem W/O-Emulgator (B1) auf der Basis von Sorbitanisostearat und einem W/O- Stabilisator (B2) auf der Basis von Polyalkylenglykol-Copolymerisat und
(C) einem W/O-Emulsionssystem auf der Basis von Methoxy-Polyalkylenglykol-Copolymerisat, wobei entweder
(1) der W/O-Emulgator von (A) verwendet wird, oder
(2) der W/O-Emulgator (B1) zusammen mit dem W/O-Stabilisator B2 (B) verwendet wird. oder
(3) eine Kombination, bestehend aus den Emulgatoren (A) und (B) mit (B1) und (B2), verwendet wird, wobei jeweils
(4) zusammen mit (1) oder (2) oder (3) noch das Emulsionssystem (C) verwendet werden kann, und Wasser sowie gegebenenfalls Zusatzstoffe oder Hilfsstoffe enthalten.

2. W/O-Lotione gemäß Anspruch 1, **dadurch gekennzeichnet, daß** sie das Emulsionssystem (C) enthalten.

3. W/O-Lotionen gemäß Anspruch 1, **dadurch gekennzeichnet, daß** die Fettsäuren der Fettsäurederivate einfach oder mehrfach ungesättigte Carbonsäuren mit 16 - 24 Kohenstoffatomen sind.

4. W/O-Lotion gemäß Anspruch 1, **dadurch gekennzeichnet, daß** die Fettsäure Gammalinolensäure ist.

5. W/O-Lotion gemäß Anspruch 1, **dadurch gekennzeichnet, daß** die Derivate Triglyceride oder Ester sind.

6. W/O-Lotion gemäß Anspruch 1, **dadurch gekennzeichnet, daß** sie ein Antioxidans und/oder einen Komplexbildner enthält.

## Claims

1. W/O lotions having a content of at least one. unsaturated fatty acid derivative, which are **characterized in that** they comprise W/O emulsifiers, W/O stabilizers and/or W/O emulsion systems chosen from the group consisting of
(A) a W/O emulsifier based on ethoxylated castor oil having seven ethylene oxide units,
(B) a W/O emulsifier (B1) based on sorbitan isostearate and a W/O stabilizer (B2) based on a polyalkylene glycol copolymer and
(C) a W/O emulsion system based on a methoxy-polyalkylene glycol copolymer, either
(1) the W/O emulsifier of (A) being used, or
(2) the W/O emulsifier (B1) being used together with the W/O stabilizer B2 (B), or
(3) a combination comprising emulsifiers (A) and (B) with (B1) and (B2) being used, where, in each case,
(4) the emulsion system (C) can also be used together with (1) or (2) or (3), and water and, if appropriate, additives or auxiliaries.

2. W/O lotions according to Claim 1, **characterized in that** they comprise emulsion system (C).

3. W/O lotions according to Claim 1, **characterized in that** the fatty acids of the fatty acid derivatives are mono- or polyunsaturated carboxylic acids having 16 - 24 carbon atoms.

4. W/O lotion according to Claim 1, **characterized in that** the fatty acid is gamma-linolenic acid.

5. W/O lotion according to Claim 1, **characterized in that** the derivatives are triglycerides or esters.

6. W/O lotion according to Claim 1, **characterized in that** it comprises an antioxidant and/or a complexing agent.

## Revendications

1. Lotions E/H contenant au moins un dérivé d'acide gras insaturé, qui sont **caractérisées en ce qu'**elles comprennent des émulsifiants E/H, des stabilisants E/H et/ou des systèmes d'émulsion E/H choisis parmi le groupe constitué de
A) un émulsifiant E/H à base d'huile de ricin éthoxylée renfermant sept motifs oxyde d'éthylène,
(B) un émulsifiant E/H (B1) à base d'isostéarate de sorbitane et un stabilisant E/H (B2) à base d'un copolymère de polyalkylèneglycol et
(C) un système d'émulsion E/H à base d'un copolymère de méthoxy-polyalkylèneglycol, en utilisant soit
(1) l'émulsifiant E/H de (A), soit
(2) l'émulsifiant E/H (B1) conjointement avec le stabilisant E/H B2 (B), soit
(3) une combinaison constituée des émulsifiants (A) et (B) avec (B1) et (B2), où dans chaque cas
(4) le système d'émulsion (C) peut être utilisé conjointement avec (1) ou (2) ou (3), et de l'eau et éventuellement des additifs ou auxiliaires.

2. Lotions E/H selon la revendication 1, **caractérisées en ce qu'**elles comprennent le système d'émulsion (C).

3. Lotions E/H selon la revendication 1, **caractérisées en ce que** les acides gras des dérivés d'acides gras sont des acides carboxyliques mono- ou polyinsaturés ayant de 16 à 24 atomes de carbone.

4. Lotion E/H selon la revendication 1, **caractérisée en ce que** l'acide gras est l'acide gammalinolénique.

5. Lotion E/H selon la revendication 1, **caractérisée en ce que** les dérivés sont des triglycérides ou des esters.

6. Lotion E/H selon la revendication 1, **caractérisée en ce qu'**elle comprend un antioxydant et/ou un agent complexant.
